# EUROPEAN PATENT SPECIFICATION

(11) **EP 0 630 252 B1**
(45) Date of publication and mention of the grant of the patent: **15.12.2004**
(21) Application number: 93914523.1
(22) Date of filing: 04.02.1993
(51) Int. Cl.: A61K 31/70, A61P 17/00

(54) **PHARMACEUTICAL PREPARATION BASED ON RHAMNOLIPID AGAINST DERMATOLOGICAL DISEASES**
PHARMAZEUTISCHE ZUBEREITUNG AUF DER BASIS VON RHAMNOLIPID GEGEN DERMATOLOGISCHE ERKRANKUNGEN
PREPARATION PHARMACEUTIQUE A BASE DE RHAMNOLIPIDES UTILISEE POUR TRAITER LES MALADIES DERMATOLOGIQUES

(30) Priority: 04.02.1992 BE 9200115; 10.04.1992 US 866691
(43) Date of publication of application: 28.12.1994
(73) Proprietor: Paradigm Biomedical Inc., New York, NY 10011-1101 (US)
(72) Inventor: Piljac, Goran, Davis, CA 95616 (US); Piljac, Visnja, Davis, CA 95616 (US)
(74) Representative: Kröncke, Rolf, Dr.
(86) International application number: PCT/EP1993/000270
(87) International publication number: WO 1993/014767

(56) References cited:
- WO-A-90/11069
- DD-A- 248 279
- DE-A- 2 150 375
- PATENT ABSTRACTS OF JAPAN vol. 13, no. 61 (C-567)(3409) 10 February 1989
- FAT SCI. TECHNOL. vol. 91, no. 9, 1989, pages 363 - 366 S. LANG ET AL. 'Antimicrobial Effects of Biosurfactants'
- J. ANTIBIOT. vol. 24, no. 12, 1971, pages 855 - 859 S. ITOH ET AL. 'Rhamnolipids produced by Pseudomonas Aeruginosa grown on n-Paraffin'
- CURRENT MICROBIOLOGY vol. 10, no. 6, 1984, pages 323 - 328 T.R. SHRYOCK ET AL. 'Effect of Pseudomonas aeruginosa Rhamnolipid on Human Neutrophil Migration'
- DATABASE WPIL Week 8544, Derwent Publications Ltd., London, GB; AN 85-272338
- PATENT ABSTRACTS OF JAPAN vol. 12, no. 306 (C-522)(3153) 19 August 1988
- FEBS LETTERS vol. 139, no. 1, 1982, pages 81 - 85 T. HIRAYAMA ET AL. 'Novel Methyl Rhamnolipids from Pseudomonas Aeruginosa'
- ACTA BIOTECHNOLOGICA vol. 7, no. 4, 1987, pages 353 - 356 D. HAFERBURG ET AL. 'ANTIPHYTOVIRALE AKTIVIT[T VON RHAMNOLIPID AUS PSEUDOMONAS AERUGINOSA'

## Description

The present invention relates to a pharmaceutical preparation comprising as an active ingredient at least one rhamnolipid. In particular, the present invention relates to such a pharmaceutical preparation for treatment of dermatological diseases.

Among bio-surfactants are glycolipids. Due to different combinations of carbohydrates and lipids, together with structural different bonds and different ionic states, there are variety of glycolipids having mutually a strongly different hydrophilic/lipophilic balance. It is known that various strains of Pseudomonas, such as Pseudomonas aaeruginosa, Pseudomonas fluorescens, Pseudomonas putida, Pseudomonas oleovorans are capable of extra cellular secretion of rhamnolipids, when growing on soluble and insoluble carbon sources.

Patent abstracts of JAPAN, vol. 13, no. 61 10 February 1989 and JP-A-63253025 discloses the use of Rhamnolipids in the treatment of inflammatory diseases when administered orally or parenterally.

FAT. SCI. TECHNOL. vol 91, no. 9, 1989, pages 363-366, S. Lang et al: "Antimicrobial Effects of Biosurfactants" teaches that Rhamnolipids possess antimicrobial properties, specifically that they prevent the growth of Gram-positive bacteria.

J. of antibiotics 1971. vol. 24, no. 12, 1971, pages 855-859 S Itoh et al. teaches that Rhamnolipids may exhibit varying biological activities in vitro.

Current Microbiol. vol. 10. no. 6, 1984, pages 323-328, T.R. Shryock et al. discloses that Pseudomonas Rhamnolipid stimulates both chemotaxis and chemokinesis.

Febs Letters, vol. 139, no. 1, 1982, pages 81-85 T. Hirayama et al. discloses novel methyl Rhamnolipids from Pseudomonas earuginosa.

DE-A-2 150 375. discloses antiviral activity of 2-0-9-L-Rhamnopyranosyl-(-1-Rhamnopyranosyl-3-hydroxy decanoyl-3-hydroxyde-canoic acid.

Duhring, Lupus erythematosus systemicus, Herpes simplex infections, Papilloma virus infections, Ichtyosis vulgaris, Erythrodermia ichtyosiformis, Keratodermitis palmoplantaris, Epidermolysis bullosa hereditaria simplex and the like.

Preferably, the rhamnolipid is a rhamnolipid of the general formula

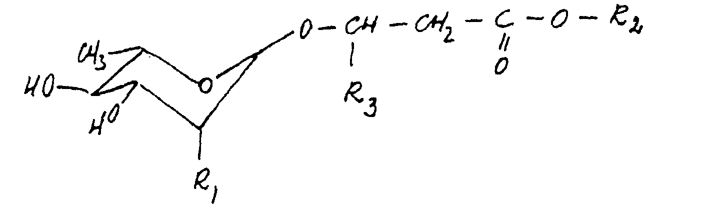

Very active rhamnolipids are obtained when the rhamnolipid is a di-rhamnolipid.

When the substituent R₂ is hydrogen, the rhamnolipid comprises only one lipid group. When the substituent R₂ is formed by the group 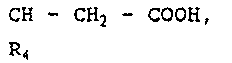 the rhamnolipid molecule comprises two lipid units mutually connected by an ester bond.

The substituents R₃ and R₄ may be selected from straight or branched (C₅-C₂₀)-saturated mono or poly-unsaturated alkyl groups. Preferred are unbranched, saturated alkyl groups having the general formula (CH₂)ₓ-CH₃, wherein x = 4-19. More preferably x = 4 or 6 for the alkyl groups.

A very active pharmaceutical preparation is obtained when it comprises the rhamnolipid is (alpha-L-rhamnopyranosyl-(1,2) alpha-L-rhamnopyranosyl)-3-hydroxydecoanoyl-3-hydroxydecanoic acid.

Based on a non-limitative example, the isolation of rhamnolipid and the use in a pharmaceutical preparation according to the present invention will be described. Herein the following methods will be used.

### 1. Isolation and characterization

From oil well drilling mud bacterial strains have been isolated, which strains can synthesize rhamnolipids both on a soluble carbon source (glucose) as on a insoluble carbon source (glycerol, gas oil). These isolated bacterial strains have been characterized as Pseudomonas strains (BBL Minitek, Numerical Determination and Identification System, Becton, Dichinson and Company).

### 2. Concentration and purification

The rhamnolipids present in the medium due to extra cellular secretion have been concentrated and purified using different procedures.

### a) acid precipitation

The pH of the supernatant was adjusted to pH 1.5-2.0 using hydrochloric acid, then it was evaporated to 1/10 of the initial volume and left over the night at 4°C. Pellet (0.5-2.0 g/l) was centrifuged at 17.300 x g for 30 minutes at 4°C and subsequently extracted using CH₂Cl₂, filtrated, evaporated, resuspended in water and precipitated again.

### b) foam fractionation

Compressed air was introduced into the supernatant and foam formed was passed into a separate container, or was introduced in acidified water (pH 1.5-2.0).

### c) chromatography

Supernatant was applied on a preparative column (Amberlit XAD-8 or XAD-2 resin, Rohm & Haas). The column was equilibrated using water, and after absorption rinsed with water. The active compounds were eluted using a lower alkyl alcohol, such as ethanol and methanol. The solvent used was evaporated under vacuum and the concentrate was acidified and precipitated as described above.

### 3. Chemical detection and characterization

Thin layer chromatography was performed analytically using silica gel 60F 254 plates (Merck), and performed preparatively using Kemika plates. For an optimal separation the following solvent mixtures may be used
CHCl₃-MeOH-acetic acid-water (25:15:4:2; 12:15:4:2; 25:25:4:2)
CHCl₃-MeOH-acetic acid (80:15:5)
Hexane-isopropyl ether-acetic acid (15:10:1)
CHCl₃-MeOH-NH₄OH (25%)-water (65:25:4:2)
propanol-NH₄OH (25%) (4:1).

### EXAMPLE

Pseudomonas aeruginosa was cultured at a temperature of 32°C. The following culture media were very suitable for rhamnolipid production.
a) 5 g glucose, 5 g peptone, 2 g yeast extract, 5 g NaCl, 0,5 g KH₂PO₄, 2 g MgSO₄.H₂O, 3 g KNO₃, 1 ml Gottlib solution and 1 liter water.
b) 20 g glucose, 10 g yeast extract, 20 g CaCO₃ and 1 liter water.

Glucose may be replaced by glycerol, whereby a two phase system is formed. As a carbon source glycerol provides a better yield in comparison to glucose, but a more complicated process is required. The change in surface tension of the culture media to 28-31 mN/m was measured using a White's ring tensiometer. A decrease of the surface tension is a good indication of the yield at the end of the fermentation. The rhamnolipid concentration was measured spectrophotometrically using anthrone reagents. After separation of the biomass by centrifugation, the supernatant was chromatographed on silica gel 60 F 254 plates and on Kemika plates.

The presence of glycolipids was proved using the following reagents, alpha naphthol for lipids, diphenylamine for glycolipids, and beta naphthol thymol and anthrone for carbohydrates and oligosaccharides. Using these reagents, it is proved that the compounds are of the glycolipid type. Using alphacyclodextrin, reagent and dye for fatty acids, it is proved that the lipophilic/hydrophilic part of the molecule consists of fatty acid chains having an even number of carbon atoms. Negative results were obtained using reagents for unsaturated lipids. Using alkali KMnO₄ and ammoniacal AgNO₃, the presence of glycosidic bonds has been proved. Using alkali hydrolysis ester bonds between fatty acids are split, and using acidic hydrolysis, the O-glucosidic bond between a sugar group and the OH group of the lipid part of the molecule is split.

10 Volumina of the supernatant comprising rhamnolipids was passed over an amberlit XAD-8 of XAD-2 column (Rohm & Haas). The column was washed with water. The rhamnolipids were eluted using 100% MeOH. The fractions comprising rhamnolipids were evaporated and subsequently added to pure water.1 N HCl was added to precipitate the rhamnolipids. Precipitated rhamnolipids were centrifuged at 3000 rpm for 10 minutes. The rhamnolipids now precipitated were washed using pure water and subsequently centrifuged at 3000 rpm for 10 minutes. Using 1/10 N NaOH the pH of the precipitated lipids were adjusted to 7.2. After lyophilization 10 g of the lyophilized preparation was dissolved in 50 ml propanol and applied to a silica column (Waters HPLC, volume 500 ml) equilibrated with hexane. Using 5 1 propanol impurities were eluted. The rhamnolipids were eluted using a solvent mixture comprising propanol-25% NH₄OH (4:1). The active fractions were evaporated and dissolved in water, again precipitated in 1 N HCl, centrifuged at 3000 rpm, and the precipitants were adjusted to pH 7.2 using 0.1 N NaOH, and lyophilized.

The pure rhamnolipids obtained were used for preparing pharmaceutical preparations according to the invention. These preparations may comprise solutions, ointment, cremes, gels, fluids, powders, pills and the like.

Dependent on the disease to be treated, such as dermatological diseases, the seriousness of the disease, the age of the patient, and the like, pharmaceutical preparations may comprise 0.05 to 10 % by weight active rhamnolipid. Preferably, the rhamnolipid concentration is 0.1 to 2.0 % by weight, more preferably 0.1 to 1.0% by weight. The under limit is determined by the activity of the present rhamnolipid and the upper limit is predominantly determined economically.

### Formulation example

A ointment base was enriched using 1.0% by weight rhamnolipid. The rhamnolipid was (alpha-L-rhamnopyranosyl-(1,2) alpha-L-rhamnopyranosyl)-3-hydroxydecoanoyl-3-hydroxydecanoic acid, and was identified using
i) ¹H and ¹³C-NMR data; and
ii) massaspectrophotometric data.

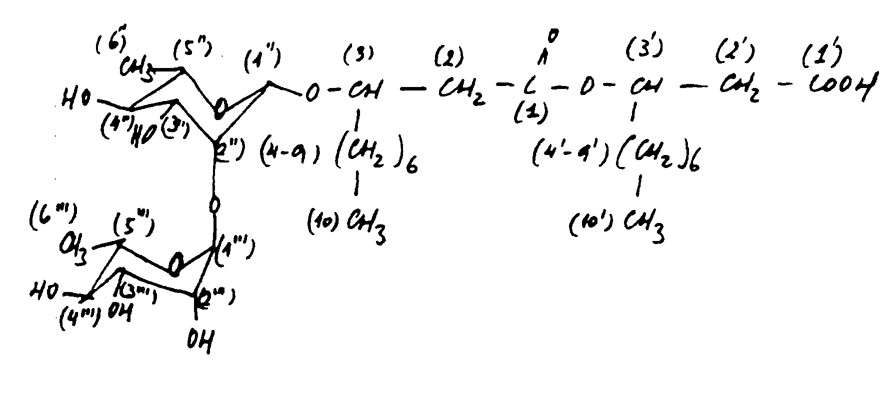

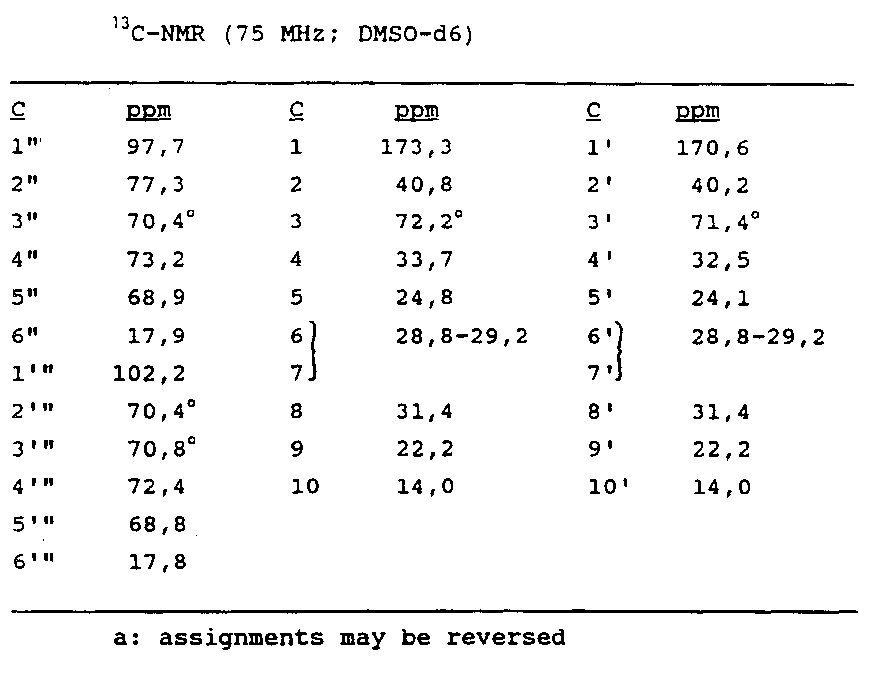

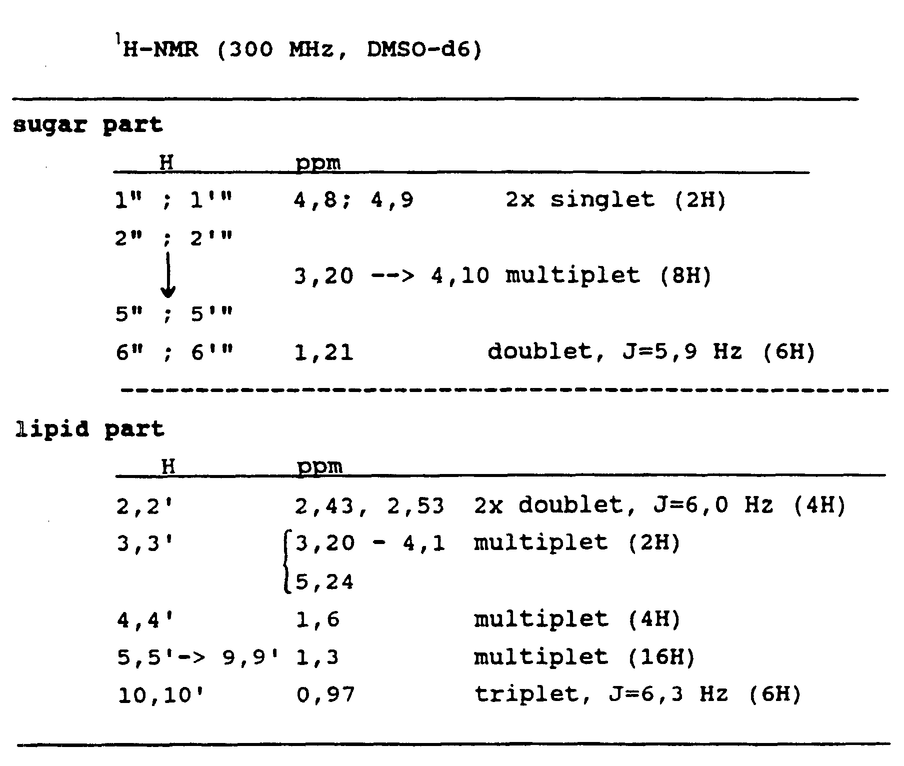

massaspectra
m/z : 673 [M + H + Na]⁻
m/z : 695 [M + H + 2Na]⁺
m/z : 525 [M - C₁₀H₁₈O₂ + 2Na]⁺ minus terminal lipid
m/z : 379 [M - C₁₀H₁₈O₂ - rhamnose + 2Na]⁺ minus terminal lipid and rhamnose

For human patient the following dermatological diseases were treated using the preparation according to the formulation example: Psoriasis pustulosa, Neurodermitis chronica multilocularis, Lichen ruber planus and Acne papulopustulosa. The treatment of affected skin surfaces for a period of about two weeks resulted in a substantially total disappearance of the features of the dermatological diseases.

## Claims

1. Use of a rhamnolipid of the general formula: 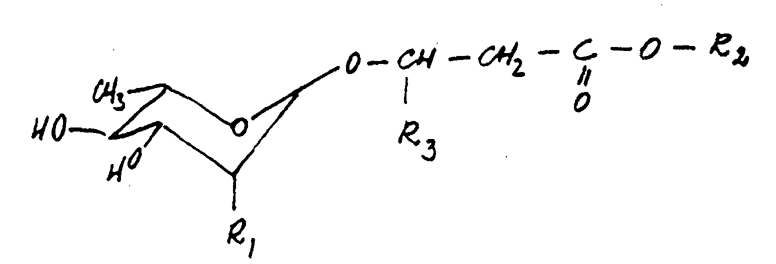 wherein
R₁ = H, alpha-L-rhamnopyranosyl; 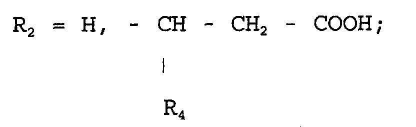 R₃ = (C₅-C₂₀)-saturated, -mono- or poly-unsaturated alkyl;
R₄ - (C₅-C₂₀)-saturated, -mono- or poly-unsaturated alkyl; for the manufacture of a medicament for treatment of Acnea vulgaris, Dermatitis allergica contactu, Neurodermitis circumscripta multilocularis, Neurodermitis diffusa, Neurodermitis erythrodermica, Neurodermitis circumscripta chronica unilocularis, Neurodermitis verrucosa, Neurodermitis infantum, Prurigo chronica, Rosacea, Psoriasis vulgaris, Lynchen ruber planus, Dermatitis nummularis eczematoides, Epydermolysis bullosa hereditaria dystrophica recessiva, Dermatitis seborrhoica, Erythema nodosum, Pemfigus vulgaris, Dermatitis herpetiformis Durhing, Lupus erythematosus systemicus, Ichtyosis vulgaris, Erythrodermis ichtyosiformis, Keratodermitis palmoplantaris, Epidermolysis bullosa hereditaria simplex.

2. Use as claimed in claim 1, wherein
R₃ = -(CH₂)ₓ -CH₃, and x = 4-19.

3. Use as claimed in claim 2, wherein
x = 4 or 6.

4. Use as claimed in claim 2 or 3, wherein
R₄ = -(CH₂)ₓ -CH₃, and x = 4-19.

5. Use as claimed in claim 4, wherein
x = 4 or 6.

6. Use as claimed in claims 1-5, wherein
R₁ = alpha-L-rhamnopyranosyl.

7. Use as claimed in claim 2-6, wherein x = 6.

8. Use as claimed in claim 1-7, wherein 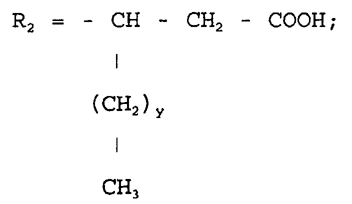 and y = 4-19.

9. Use as claimed in claim 8, wherein
Y = 4 or 6.

10. Use as claimed in claim 1-9, wherein the rhamnolipid is (alpha-L-rhamnopyranosyl-(1,2) alpha-L-rhamnopyranosyl)-3-hydroxydecoanoyl-3-hydroxydecanoic acid.

11. Use according to claim 10, for the manufacture of a medicament for the treatment of psoriasis.

12. Use according to claim 10 for the manufacture of a medicament for the treatment of lichen ruber planus dermatitis allergica contactu, dermatitis numularis eczematoides, neurodermatitis circumscripta multilocularis and ichthyosis vulgaris.

13. Use according to any of the preceding claims wherein the medicament has a rhamnolipid concentration of 0.05 to 10% weight active rhamnolipid.

## Patentansprüche

1. Verwendung eines Rhamnolipids der allgemeinen Formel: 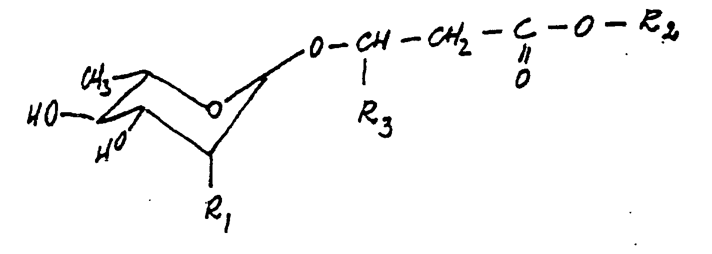 mit
R₁ = H, Alpha-L-Rhamnopyranosyl; 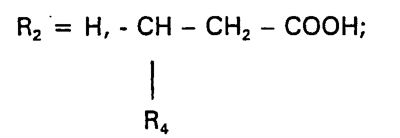 R₃ = (C₅-C₂₀)-gesättigtes, -mono- oder poly-ungesättigtes Alkyl;
R₄ = (C₅-C₂₀)-gesättigtes; -mono- oder poly-ungesättigtes Alkyl; zur Herstellung eines Medikaments zur Behandlung von Acnea Vulgaris, Dermatitis allergica contactu, Neurodermitis circumscripta multilocularis, Neurodermitis diffusa, Neurodermitis erythrodermica, Neurodermitis circumscripta chronica unilocularis, Neurodermitis verrucosa, Neurodermitis infantum, Prurigo chronica, Rosacea, Psoriasis vulgaris, Lynchen ruber planus, Dermatitis nummularis eczematoides, Epydermolysis bullosa hereditaria dystrophica recessiva, Dermatitis seborrhoica, Erythema nodosum, Pemfigus vulgaris, Dermatitis herpetiformis Durhing, Lupus erythematosus systemicus, lchtyosis vulgaris, Erthrodermis ichtyosiformis, Keratodermitits palmoplantaris, Epidermolysis bullosa hereditaria simplex.

2. Verwendung gemäß Anspruch 1, wobei
R₃ = - (CH₂)ₓ -CH₃, und x = 4-19 ist.

3. Verwendung gemäß Anspruch 2, wobei
x = 4 oder 6 ist.

4. Verwendung gemäß Anspruch 2 oder 3, wobei
R₄ = -(CH₂)ₓ -CH₃, und x = 4-19 ist.

5. Verwendung gemäß Anspruch 4, wobei
x = 4 oder 6 ist.

6. Verwendung gemäß einem der Ansprüche 1 bis 5, wobei
R, = Alpha-L-Rhamnopyranosyl ist.

7. Verwendung gemäß einem der Ansprüche 2 bis 6, wobei
x = 6 ist.

8. Verwendung gemäß einem der Ansprüche 1 bis 7, wobei 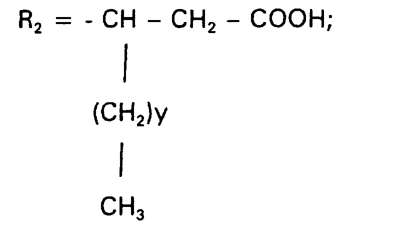 und y = 4-19 ist.

9. Verwendung gemäß Anspruch 8, wobei
y = 4 oder 6 ist.

10. Verwendung gemäß einem der Ansprüche 1 bis 9, wobei das Rhamnolipid (Alpha-L-Rhamnopyranosyl-(1,2) Alpha-L-rhamnopyranosyl)-3-Hydroxydecoanoyl-3-Hydroxydecansäure ist.

11. Verwendung gemäß Anspruch 10 zur Herstellung eines Medikaments zur Behandlung von Psoriasis.

12. Verwendung gemäß Anspruch 10 zur Herstellung eines Medikaments zur Behandlung von Lichen Ruber planus Dermatitis allergica contactu, Dermatitis numularis eczematoides, Neurodermatitis circumscripta multilocularis und lchthyosis vulgaris.

13. Verwendung gemäß einem der vorherigen Ansprüche, wobei das Medikament eine Rhamnolipidkonzentration von 0,05 bis 10 Gew.% wirksamen Rhamnolipid aufweist.

## Revendications

1. Utilisation d'un rhamnolipide de formule générale : 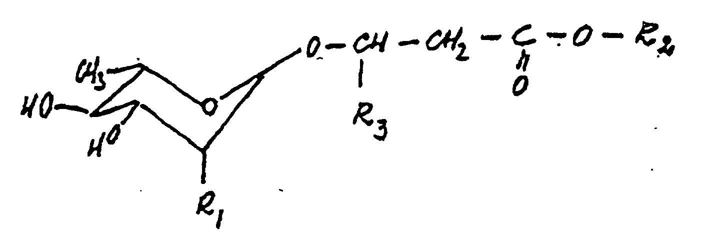 dans laquelle
R₁ = H, alpha-L-rhamnopyranosyl ; 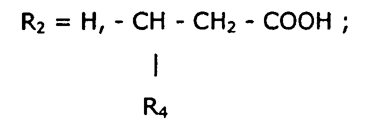 R₃ = alkyle en (C₅-C₂₀)-saturé, -mono- ou poly-insaturé ;
R₄ = alkyle en (C₅-C₂₀)-saturé, -mono- ou poly-insaturé ; pour la fabrication d'un médicament pour le traitement de Acnea vulgaris, Dermatitis allergica contactu, Neurodermitis circumscripta multilocularis, Neurodermitis diffusa, Neurodermitis erythrodermica, neurodermitis circumscripta chronica unilocularis, Neurodermitis verrucosa, Neurodermitis infantum, Prurigo chronica, Rosacea, Psoriasis vulgaris, Lynchen ruber planus, Dermatitis nummularis eczematoides, Epydermolysis bullosa hereditaria dystrophica recessiva, Dermatitis seborrhoica, Erythema nodosum, Pemfigus vulgaris, Dermatitis herpetiformis Durhing, Lupus erythematosus systemicus, Ichtyosis vulgaris, Erythrodermis ichtyosiformis, Keratodermitis palmoplantaris, Epidermolysis bullosa hereditaria simplex.

2. Utilisation selon la revendication 1, dans laquelle
R₃ = - (CH₂)ₓ - CH₃, et x= 4-19.

3. Utilisation selon la revendication 2, dans laquelle
x = 4 ou 6.

4. Utilisation selon la revendication 2 ou 3, dans laquelle
R₄ = - (CH₂)ₓ - CH₃, et x= 4-19.

5. Utilisation selon la revendication 4, dans laquelle
x = 4 ou 6.

6. Utilisation selon les revendications 1-5, dans laquelle
R₁ = alpha-L-rhamnopyronosyl

7. Utilisation selon la revendication 2-6, dans laquelle x = 6.

8. Utilisation selon la revendication 1-7, dans laquelle 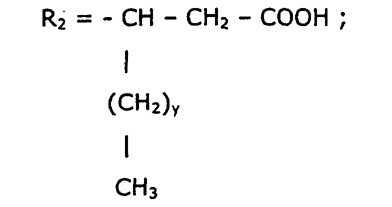 et y= 4-19.

9. Utilisation selon la revendication 8, dans laquelle
y = 4 ou 6.

10. Utilisation selon la revendication 1-9, dans laquelle le rhamnolipide est l'acide (alpha-L-rhamnopyranosyl-(1,2) alpha-L-rhamnopyranosyl)-3-hydroxydécanoyle-3-hyroxydécanoïque.

11. Utilisation selon la revendication 10, pour la fabrication d'un médicament pour le traitement de psoriasis.

12. Utilisation selon la revendication 10, pour la fabrication d'un médicament pour le traitement de lichen ruber planus dermatitis allergica contactu, dermatitis numularis eczematoides, neurodermatitis circumscripta multilocularis et de ichthyosis vulgaris.

13. Utilisation selon l'une des revendications précédentes, dans laquelle le médicament présente une concentration en rhamnolipide de 0,05 à 10% en poids de rhamnolipide actif.
